# EUROPEAN PATENT APPLICATION

(11) **EP 1 941 929 A1**
(43) Date of publication of application: **09.07.2008**
(21) Application number: 06811032.9
(22) Date of filing: 02.10.2006
(51) Int. Cl.: A61N 1/30

(54) **ELECTRODE STRUCTURE FOR IONTOPHORESIS COMPRISING SHAPE MEMORY SEPARATOR, AND IONTOPHORESIS APPARATUS COMPRISING THE SAME**

(30) Priority: 30.09.2005 JP 2005288618
(71) Applicant: TTI ELLEBEAU, INC., Tokyo 140-0002 (JP)
(72) Inventor: TANIOKA, Akihiko, Tokyo 145-0061 (JP); NAKAYAMA, Mizuo c/o TTI ellebeau, Inc., Tokyo 150-0022 (JP); MATSUMURA, Takehiko c/o TTI ellebeau, Inc., Tokyo 150-0022 (JP); AKIYAMA, Hidero c/o TTI ellebeau, Inc., Tokyo 150-0022 (JP); MATSUMURA, Akihiko c/oTTI ellebeau, Inc., Tokyo 150-0022 (JP)
(74) Representative: Liesegang, Eva
(86) International application number: PCT/JP2006/319684
(87) International publication number: WO 2007/037475

(57) **Abstract**

The present invention relates to an electrode assembly for iontophoresis including a separator preventing the movement of a substance between an electrolyte solution and a drug solution until the electrode assembly for iontophoresis is used and enabling the movement of a certain substance at the time of use. More specifically, the present invention relates to an electrode assembly for iontophoresis which includes at least: an electrode connected to an electric power source device having the same polarity as that of the ionic drug in the electrode assembly; an electrolyte solution holding portion impregnated with the electrolyte solution, the electrolyte solution holding portion being placed adjacent to the electrode; an ion exchange membrane which is selectively permeable to an ion having a polarity opposite to that of the ionic drug, the ion exchange membrane being placed adjacent to the electrolyte solution holding portion; a drug solution holding portion impregnated with the ionic drug, the drug solution holding portion being placed adjacent to the ion exchange membrane; and an ion exchange membrane which is selectively permeable to an ion having the same polarity as that of the ionic drug, the ion exchange membrane being placed adjacent to the drug solution holding portion, wherein a shape-memory separator capable of switching between passage of a substance and blocking of the substance owing to deformation of a shape-memory resin is placed adjacent to at least one surface of the ion exchange membrane which is selectively permeable to an ion having a polarity opposite to that of the ionic drug.

## Description

### CROSS-REFERENCE TO RELATED APPLICATIONS

This application claims the benefit of priority on the basis of the prior Japanese Patent Application No. 2005-288618 (filed on date: September 30, 2005), the entire disclosure of which is incorporated herein by reference.

### BACKGROUND OF THE INVENTION

### Field of Invention

The present invention relates to a technique (transdermal drug delivery) for transdermally administering various ionic drugs by means of iontophoresis. More specifically, the present invention relates to an electrode assembly to be used for iontophoresis and an iontophoresis device using the same.

### Background Art

A method of introducing (permeating) an ionic drug placed on the surface of the skin or mucosa (hereinafter, merely referred to as "skin") of a predetermined site of a living body into the body through the skin by giving the skin an electromotive force sufficient to drive such an ionic drug is called iontophoresis (iontophorese, ion introduction method, ion permeation therapy) (see, for example, JP 63-35266 A).

For example, positively charged ions are driven (transported) into the skin on the side of an anode (positive electrode) in an electric system of an iontophoresis device. On the other hand, negatively charged ions are driven (transported) into the skin on the side of a cathode (negative electrode) in the electric system of the iontophoresis device.

A large number of such iontophoresis devices as described above have been conventionally proposed (see, for example, JP 63-35266 A, JP 04-297277 A, JP 2000-229128 A, JP 2000-229129 A, JP 2000-237327 A, JP 2000-237328 A and WO 03/037425 A1). Some of those documents each propose, as an electrode assembly for iontophoresis, the electrode assembly obtained by laminating: an electrode; an electrolyte solution holding portion; an ion exchange membrane which is selectively permeable to an ion having polarity opposite to that of an ionic drug; a drug solution holding portion comprising the ionic drug impregnated with the ionic drug; and an ion exchange membrane which is selectively permeable to an ion having the same polarity as that of the ionic drug.

However, during the period from the end of the production of the electrode assembly until the use of the assembly, a component in the electrolyte solution or a component in the drug solution (mainly an ion component having polarity opposite to that of the ionic drug) may move through the ion exchange membrane which is selectively permeable to an ion having polarity opposite to that of the ionic drug. A certain component in the electrolyte solution or a certain component in the drug solution may cause an adverse effect (such as the alteration of a drug component, a reduction in stability of the drug, a reduction in amount of the drug that can be released, or a reduction in transport number due to the mixing of a dissimilar ion).

Therefore, preventing the movement of a substance between the electrolyte solution and the drug solution until the electrode assembly for iontophoresis is used, and enabling the movement of a certain substance at the time of use are important problems.

### SUMMARY OF THE INVENTION

The present invention has been made in view of the above-described problems of the prior art, and an object of the present invention is to provide an electrode assembly for iontophoresis comprising a separator preventing the movement of a substance between an electrolyte solution and a drug solution until the electrode assembly for iontophoresis is used and enabling the movement of a certain substance at the time of use, and an iontophoresis device using the same.

To solve the above problems, an electrode assembly for iontophoresis comprising an ionic drug according to the present invention includes at least: an electrode connected to an electric power source device having the same polarity as that of the ionic drug in the electrode assembly; an electrolyte solution holding portion impregnated with the electrolyte solution, the electrolyte solution holding portion being placed adjacent to the electrode; an ion exchange membrane which is selectively permeable to an ion having a polarity opposite to that of the ionic drug, the ion exchange membrane being placed adjacent to the electrolyte solution holding portion; a drug solution holding portion impregnated with the ionic drug, the drug solution holding portion being placed adjacent to the ion exchange membrane; and an ion exchange membrane which is selectively permeable to an ion having the same polarity as that of the ionic drug, the ion exchange membrane being placed adjacent to the drug solution holding portion, wherein a shape-memory separator capable of switching between passage of a substance and blocking of the substance owing to deformation of a shape-memory resin is placed adjacent to at least one surface of the ion exchange membrane which is selectively permeable to an ion having a polarity opposite to that of the ionic drug.

In a preferred aspect of the present invention, the separator is made of a membrane of the shape-memory resin capable of being porous or a porous membrane containing the shape-memory resin, and the separator closes a pore to block movement of a substance during production or storage of the electrode assembly, and changes to be porous owing to a temperature change or voltage application, thereby allowing the substance to pass.

In other preferred aspect of the present invention, the separator blocks the passage of the substance at a temperature lower than 30°C and becomes porous owing to deformation of the shape-memory resin by heating at 30°C or higher, thereby allowing the substance to pass.

In another preferred aspect of the present invention, the separator becomes porous owing to deformation of the shape-memory resin by heating at 40°C or higher, thereby allowing the substance to pass, and then maintains a deformed state even when being cooled to a temperature lower than 40°C to thereby allow the substance to pass.

Also, an iontophoresis device according to the present invention includes: an electric power source device; a drug administration means comprising two or more electrode assemblies including one or more electrode assemblies comprising the ionic drug; and a current control means for controlling a current flowing to the electrode assemblies, wherein an ionic drug is released from the electrode assemblies to be administered to a living body transdermally in accordance with the current flowing from the current control means.

Another electrode assembly for iontophoresis comprising an ionic drug according to the present invention comprises at least: an electrode connected to an electric power source device having the same polarity as that of the ionic drug in the electrode assembly; an electrolyte solution holding portion impregnated with the electrolyte solution, the electrolyte solution holding portion being placed adjacent to the electrode; an ion exchange membrane which is selectively permeable to an ion having a polarity opposite to that of the ionic drug, the ion exchange membrane being placed adjacent to the electrolyte solution holding portion; a drug solution holding portion impregnated with the ionic drug, the drug solution holding portion being placed adjacent to the ion exchange membrane; and an ion exchange membrane which is selectively permeable to an ion having the same polarity as that of the ionic drug, the ion exchange membrane being placed adjacent to the drug solution holding portion, wherein a shape-memory separator capable of switching between passage of a substance and blocking of the substance owing to deformation of a shape-memory resin is placed adjacent to at least one surface of the ion exchange membrane which is selectively permeable to an ion having the same polarity as that of the ionic drug.

As described above, in the electrode assembly for iontophoresis according to the present invention, the shape-memory separator capable of switching the transmission and blocking of a substance through the deformation of the shape-memory resin is placed adjacent to at least one surface of the ion exchange membrane. As a result, the movement of a substance between the electrolyte solution and the drug solution can be prevented until the electrode assembly for iontophoresis is used, and the movement of a certain substance is enabled at the time of use.

### BRIEF DESCRIPTION OF THE DRAWINGS

Fig. 1 is a schematic view showing the outline of an electrode assembly for iontophoresis according to the present invention.
Fig. 2 is a view showing the outline of an iontophoresis device including the electrode assembly for iontophoresis according to the present invention.

### DETAILED DESCRIPTION OF THE INVENTION

Hereinafter, the present invention is described on the basis of preferable specific examples shown in the drawings.

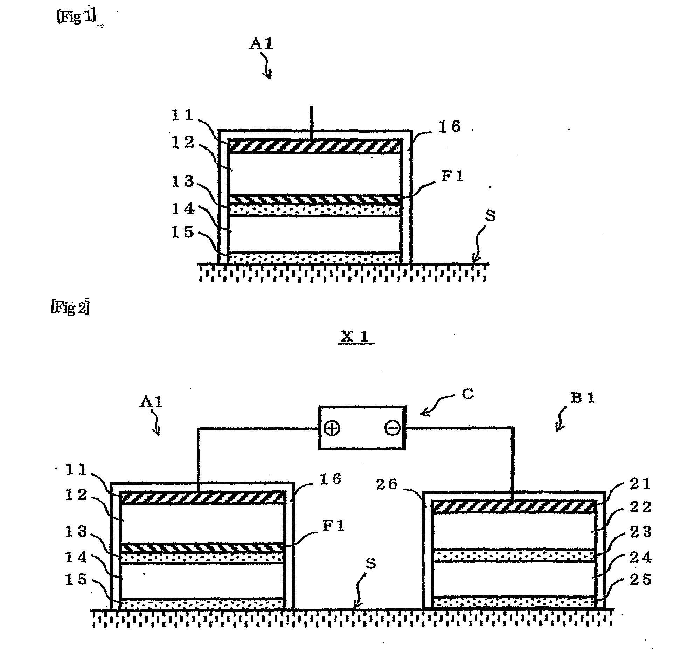

Fig. 1 is a schematic view showing a state where an electrode assembly A1 for iontophoresis according to a preferred embodiment of the present invention which is arranged on a skin S is used. The electrode assembly A1 is used as a working electrode assembly for transdermally administering an ionic drug in an iontophoresis device. The electrode assembly A1 for iontophoresis includes: an electrode 11 connected to an electric power source device having the same polarity as that of the charge of an ionic drug through an electric cable; an electrolyte solution holding portion 12 impregnated with the electrolyte solution, the electrolyte solution holding portion 12 being placed adjacent to the electrode 11; a shape-memory separator F1 placed adjacent to the electrolyte solution holding portion 12; an ion exchange membrane 13 which is selectively permeable to an ion having polarity opposite to that of the ionic drug, the ion exchange membrane 13 being placed adjacent to the separator F1; a drug solution holding portion 14 impregnated with the ionic drug, the drug solution holding portion 14 being placed adjacent to the ion exchange membrane 13; and an ion exchange membrane 15 which is selectively permeable to an ion having the same polarity as that of the ionic drug, the ion exchange membrane 15 being placed adjacent to the drug solution holding portion 14, and all these components are housed in a cover 16.

Fig. 2 is a schematic view showing a state where an iontophoresis device X1 including: the electrode assembly (working electrode assembly) A1 for iontophoresis according to the preferred embodiment of the present invention; an electric power source device C; and a non-working electrode assembly B1 as a counter electrode of the electrode assembly A1 for iontophoresis is arranged on the skin S.

The electrode assembly A1 for iontophoresis is connected to the same polarity of the electric power source device C as that of an ionic drug via an electric wire. In addition, the non-working electrode assembly B1 includes: an electrode 21 connected to the polarity of the electric power source device C opposite to that of the ionic drug via an electric wire; an electrolyte solution holding portion 22 impregnated with the electrolyte solution, the electrolyte solution holding portion 22 being placed adjacent to the electrode 21; an ion exchange membrane 23 which is selectively permeable to an ion having the same polarity as that of the ionic drug, the ion exchange membrane 23 being placed adjacent to the electrolyte solution holding portion 22; an electrolyte solution holding portion 24 impregnated with the electrolyte solution, the electrolyte solution holding portion 24 being placed adjacent to the ion exchange membrane 23; and an ion exchange membrane 25 which is selectively permeable to an ion having polarity opposite to that of the ionic drug, the ion exchange membrane 25 being placed adjacent to the electrolyte solution holding portion 24. The whole components are housed in a cover 16. The above non-working electrode assembly B1 is exemplified as one preferred embodiment, and is not limited to the above embodiment. In Fig. 2, the electrode assembly A1 for iontophoresis is connected to the + side of the electric power source device C while the non-working electrode assembly B1 is connected to the - side of the electric power source device C. Of course, the assembly A1 and the assembly B1 may be connected to the - side and the + side, respectively, depending on the polarity of the ionic drug.

In the iontophoresis device X1, when an electrode assembly A1 comprising an ionic drug is energized by the electric power source C, the ionic drug moves to a side opposite to an electrode as a result of electrophoresis by virtue of an electric field, and is transdermally administered to a living body via the ion exchange membrane 15. At this time, the ion exchange membrane 13 placed on the electrode side selects an ion having polarity opposite to that of the ionic drug, thereby preventing the movement of the ionic drug to the electrode side. Meanwhile, the ion exchange membrane 15 placed on the skin selects an ion having the same polarity as that of the ionic drug. As a result, the ionic drug can be efficiently released, whereby the ionic drug can be administered to the skin S at a high transport number. Furthermore, the electrode assembly in the present invention has such constitution as that described above, so damage to the skin based on an electrochemical reaction can be prevented and the ionic drug can be safely administered. In addition, for example, the following conditions are adopted as preferred energizing conditions in the iontophoresis device: a constant current condition, specifically, 0.1 to 0.5 mA/cm², preferably 0.1 to 0.3 mA/cm², and a safe voltage condition that realizes the above constant current, specifically, 50 V or less, preferably 30 V or less.

In the present invention, multiple working electrode assemblies or multiple non-working electrode assemblies may be included. In such case, one working electrode assembly may be caused to hold multiple kinds of ionic drugs. When multiple ionic drugs different from each other in polarity are to be administered, a working electrode assembly and a non-working electrode assembly may be arranged on an anode side, and a working electrode assembly and a non-working electrode assembly may be arranged also on a cathode side.

Alternatively, multiple electrode assemblies may be constituted as drug administering means and assembled in one package to achieve, for example, convenience of handling. A material to be used for the package in this case is not particularly limited as long as it does not affect the administration of an ionic drug, and an example of such material includes polyolefin for medical equipment. Furthermore, current control means may be arranged for surely administering a predetermined amount of a drug within a predetermined time period. The drug administering means, the current control means, and an electric power source device may be integrally constituted by constituting the electric power source device as, for example, a button battery and the current control means as, for example, an integrated circuit for miniaturization.

The separator to be used for the electrode assembly is a shape-memory separator capable of switching the transmission and blocking of a substance through the deformation of a shape-memory resin, and is placed adjacent to at least one surface of the ion exchange membrane which is selectively permeable to ion having polarity opposite to that of the ionic drug. In Fig. 1, the separator F1 is placed adjacent to the side of the electrolyte solution holding portion 12 of the ion exchange membrane 13. Alternatively, a separator may be placed on the side of the drug solution holding portion 14, or two separators may be placed on both sides.

In another embodiment of the present invention, a shape-memory separator can be placed adjacent to at least one surface of the ion exchange membrane which is selectively permeable to an ion having the same polarity as that of the ionic drug. With such constitution, the administration itself of a drug can be controlled with the separator, the passage of the drug can be permitted only when the passage is needed, and the efficient administration of the drug can be realized with improved sureness.

The term "shape-memory resin (shape-memory polymer)" as used herein typically refers to, for example, a resin which can be deformed and processed in a predetermined temperature range (temperatures equal to or higher than a glass transition temperature), which is immobilized at low temperatures, and which can return to its original shape when heated again to the predetermined temperature range (temperatures equal to or higher than the glass transition temperature). In actuality, some degree of shape-memory property is inherent in any one of most polymer materials.

The separator is preferably formed of: the membrane of a shape-memory resin that can serve as a porous body; or a porous membrane containing a shape-memory resin. Upon production or storage of the electrode assembly, the pores of the separator are closed so that the movement of a substance is blocked. The separator deforms to a porous body owing to a certain stimulus to permit the transmission of the substance. The substance whose transmission is permitted has only to contain at least an ion supposed to pass through an ion exchange membrane, and any other substance may pass. Arranging such separator can prevent the movement of a substance between the electrolyte solution and the drug solution until the electrode assembly for iontophoresis is used, and can enable the movement of the substance at the time of use. Accordingly, it becomes possible to prevent an adverse effect due to the movement of a component in the electrolyte solution or a component in the drug solution (mainly an ion component having polarity opposite to that of the ionic drug) through the ion exchange membrane during the period from the end of the production of the assembly until the use of the assembly.

Examples of the above-described certain stimulus for deforming the shape-memory resin include heat (temperature) and an electric stimulus. For example, in the case where a shape-memory resin which blocks the transmission of a substance at a temperature lower than 30°C and which deforms to be porous when heated to 30°C or higher, thereby permitting the transmission of the substance, is used, a separator can be obtained, which blocks the transmission of a substance while the electrode assembly is stored in a cold space, and which, when the electrode assembly is mounted on a living body, causes the substance to transmit by being heated with the body temperature. In addition, for example, in the case where a shape-memory resin which blocks the transmission of a substance when no voltage is applied and which deforms to be porous owing to the application of a voltage to thereby permit the transmission of the substance is used, a separator can be obtained, which blocks the transmission of a substance while the electrode assembly is stored, and which causes the substance to transmit when a voltage is applied with a view to starting the administration of a drug by means of iontophoresis.

The deformation of the shape-memory resin due to the certain stimulus may be reversible or irreversible. When a shape-memory resin that irreversibly deforms is used, the transmission of a substance through a separator can be permitted by applying once a stimulus too strong to be applicable upon mounting of the electrode assembly on a living body as long as a constituent of the electrode assembly such as a drug can withstand the stimulus. For example, the transmission of a substance through a separator is permitted by heating the separator up to 40°C once immediately before use, or the transmission of a substance through the separator is permitted by applying a voltage of 100 V to the separator once immediately before use. After that, mounting the electrode assembly on a living body allows the administration of a drug by means of iontophoresis to start. When a shape-memory resin that reversibly deforms is used, the transmission of a substance through a separator can be permitted only upon administration in the case where a drug is intermittently administered.

Any shape-memory resin can be used for the separator in the present invention without any particular limitation as long as the resin restores its shape under a predetermined condition. Examples of such resin include polyester, polyurethane, styrene·butadiene, polynorbornene, transpolyisoprene, poly N-isopropylacryalmide, and an ethylene glycol-propylene glycol copolymer.

The separator is, for example, of 1 µm to 1 mm in thickness and 0.01 µm to 100 µm in pore size. Upon formation of a shape-memory separator, for example, after a granular shape-memory resin has been subjected to compression or the like to provide a porous material, or after a shape-memory resin has been foamed to provide a porous material, the resultant porous material is compressed in a predetermined temperature range so that continuous air bubbles disappear. As a result, the resultant blocks the transmission of a substance at a temperature equal to or lower than a predetermined temperature because the resultant has no continuous air bubbles, but returns to a porous material having continuous air bubbles when heated to a temperature equal to or higher than the predetermined temperature, to thereby permit the transmission of the substance.

A specific example of such shape-memory separator includes such porous film as described in AlChE Journal Vol. 49, No. 4, p.896 to 909, that is, a polyethylene porous film having a thickness of 100 µm, a pore size of 0.28 µm, and a porosity of 69% to which graft poly(N-isopropylacrylamide) (PNIPAM) as a temperature-responsive polymer is caused to adhere by means of a plasma·graft pore filling polymerization method so that transmittance is controlled in terms of temperature.

An inactive electrode made of a conductive material such as carbon or platinum can be preferably used as the electrode of the electrode assembly.

The electrolyte solution holding portion used for the electrode assembly can be constituted by a thin film that has the property of holding an electrolyte solution by being impregnated with the electrolyte solution. The thin film can be made of the same material as that used for a drug solution holding portion for holding an ionic drug by being impregnated with the ionic drug to be described later. A desired one can be appropriately used as the electrolyte solution depending upon the conditions such as a drug to be applied. However, an electrolyte solution that damages the skin of a living body owing to an electrode reaction should be avoided. An organic acid or a salt thereof present in a metabolic cycle of a living body is preferable as the electrolyte solution in the present invention in terms of harmlessness. For example, lactic acid and fumaric acid are preferable. Specifically, an aqueous solution of 1M of lactic acid and 1M of sodium fumarate (1 : 1) is preferable. Such electrolyte solution is preferable because: it has high solubility with respect to water and passes a current well; and in the case where a current is allowed to flow at a constant level, the electric resistance is low and a change in pH is relatively small in an electric power source device.

In general, an electrolyte solution that does not interact with a drug is preferable. However, the present invention is excellent because even an electrolyte solution that causes an interaction with a drug such as the alteration of a drug component, a reduction in stability of the drug, a reduction in amount of the drug that can be released, or a reduction in transport number due to the mixing of a dissimilar ion can be suitably used.

The drug solution holding portion is constituted by a thin film that holds an ionic drug or the like by being impregnated with the ionic drug or the like. It is important for such thin film to have a sufficient ability of holding an ionic drug or the like by being impregnated with the ionic drug or the like, and a sufficient ability (ion transferability, ion conductivity) of moving an ionic drug impregnated into and held by the thin film to the skin side under predetermined electric field conditions. Examples of a material that brings together good property of holding a drug by being impregnated with the drug and good ion conductivity include hydrogel forms of acrylic resins (acrylic hydrogel film), a segmented polyurethane-based gel film, and an ion-conductive porous sheet for forming a gel-like solid electrolyte (such as a porous polymer disclosed in JP 11-273452 A using, as a base, an acrylonitrile copolymer containing 50 mol% or more, preferably 70 to 98 mol% or more of acrylonitrile and having a porosity of 20 to 80%). When such drug solution holding portion as described above is impregnated with a drug, an impregnation rate (defined by 100×(W-D)/D [%]) where D indicates a dry weight and W indicates a weight after impregnation) is preferably 30 to 40%.

A cation exchange membrane and an anion exchange membrane are preferably used together as ion exchange membranes to be used for an electrode assembly. Preferable examples of the cation exchange membrane include NEOSEPTAs (CM-1, CM-2, CMX, CMS, CMB, and CLE04-2) manufactured by Tokuyama Co., Ltd. Preferable examples of the anion exchange membrane include NEOSEPTAs (AM-1, AM-3, AMX, AHA, ACH, ACS, ALE04-2, and AIP-21) manufactured by Tokuyama Co., Ltd. Other preferable examples include: an ion exchange membrane that includes a porous film having cavities a part or whole of which are filled with an ion exchange resin having a cation exchange function; and an ion exchange membrane that includes a porous film having cavities a part or whole of which are filled with an ion exchange resin having an anion exchange function.

Here, the above-mentioned ion exchange resins can be fluorine-based ones that include a perfluorocarbon skeleton having an ion exchange group and hydrocarbon-based ones that include a nonfluorinated resin as a skeleton. From the viewpoint of convenience of production process, hydrocarbon-based ion exchange resins are preferably used. The filling rate of the porous film with the ion exchange resin, which varies depending on the porosity of the porous film, can be, for example, 5 to 95 % by mass, and is preferably 10 to 90 % by mass, or more preferably 20 to 60 % by mass.

In addition, the ion exchange group in the above-mentioned ion exchange resin is not particularly limited so far as it is a functional group that generates a group having negative or positive charge in aqueous solutions. Such functional group may be present in the form of a free acid or a salt. Examples of a cation exchange group include a sulfonic group, a carboxylic acid group, and a phosphonic acid group. Of those, a sulfonic group is preferable. Examples of a counter cation for the cation exchange group include: alkali cations such as a sodium ion and a potassium ion; and ammonium ions. Examples of an anion exchange group include a primary amino group, a secondary amino group, a tertiary amino group, a quaternary amino group, a pyridyl group, an imidazole group, a quaternary pyridium group, and a quaternary imidazolium group. Of those, a quaternary ammonium group or a quaternary pyridium group is preferable. Examples of a counter cation for the anion exchange group include: halogen ions such as a chlorine ion; and hydroxy ions.

In addition, the above-mentioned porous film is not particularly limited and any porous film can be used as far as it is in the form of a film or sheet that has a large number of pores communicating both sides thereof. To satisfy both of high strength and flexibility, it is preferable that the porous film be made of a thermoplastic resin. Examples of the thermoplastic resin constituting the porous film include: polyolefin resins such as homopolymers or copolymers of α-olefins such as ethylene, propylene, 1-butene, 1-pentene, 1-hexene, 3-methyl-1-butene, 4-methyl-1-pentene, and 5-methyl-1-heptene; vinyl chloride-based resins such as polyvinyl chloride, vinyl chloride-vinyl acetate copolymers, vinyl chloride-vinylidene chloride copolymers, and vinyl chloride-olefin copolymers; fluorine-based resins such as polytetrafluoroethylene, polychlorotrifluoroethylene, polyvinylidene fluoride, tetrafluoroethylene-hexafluoropropylene copolymers, tetrafluoroethylene-perfluoroalkyl vinylether copolymers, and tetrafluoroethylene-ethylene copolymers; polyamide resins such as nylon 66; and polyimide resins. Of those, polyolefin resins are preferable in consideration of, for example, mechanical strength, flexibility, chemical stability, and chemical resistance. Of those, polyethylene or polypropylene is more preferable, and polyethylene is still more preferable.

Further, the mean pore size of the above-mentioned porous film made of the thermoplastic resin is preferably 0.005 to 5.0 µm, more preferably 0.01 to 2.0 µm, or still more preferably 0.02 to 0.2 µm. Note that, the above-mentioned mean pore size as used herein means a mean flow pore size measured in conformance with the bubble point method (JIS K3832-1990).

In addition, the porosity of the porous film is preferably 20 to 95 %, more preferably 30 to 90 %, or still more preferably 30 to 60 %. In consideration of the thickness of an ion exchange membrane to be finally formed, the thickness of the porous film is preferably 5 to 140 µm, more preferably 10 to 130 µm, or still more preferably 15 to 55 µm. An anion exchange membrane or a cation exchange membrane formed of such porous film generally has the same thickness as that of the porous film or up to about 20 µm larger than the thickness of the porous film.

As described above, the electrode assembly for iontophoresis according to the present invention is characterized in that the assembly comprises an ionic drug.

### Specific examples of the drug are as follows.

Examples of an ionic drug include: anesthetic drugs (such as procaine hydrochloride and lidocaine hydrochloride); gastrointestinal disease therapeutic (such as carnitine chloride); skeletal muscle relaxants (such as vancuronium bromide); and antibiotics (such as a tetracyciine-based preparation, a kanamycin-based preparation, and a gentamicin-based preparation).

Examples of the ionic drug that can be negatively charged include: vitamin (such as riboflavin sodium, nicotine acid, ascorbic acid, and folic acid); adrenal cortex hormones (such as a hydrocortisone-based water-soluble preparation, a dexamethasone-based water-soluble preparation, and a prednisolone-based water-soluble preparation such as prednisolone sodium phosphate and dexamethasone sodium phosphate); and antimicrobial drug (such as a quinolone-based preparation).

Examples of a vaccine include a BCG vaccine, a hepatitis A vaccine, a melanoma vaccine, a measles vaccine, a poliomyelitis vaccine, and an influenza vaccine.

Examples of an adjuvant include MPL (Monophosphoryl lipid A), DMPC (dimyristaylphosphatidylcholine), QS-21, DDA (Dimethyl dioctadecyl ammonium chloride), and RC-529.

Furthermore, examples of a preferable combination of a vaccine and an adjuvant include: a combination of a positively ionized vaccine and RC-529; a combination of a negatively ionized vaccine and DDA; a combination of a BCG vaccine and MPL; a combination of a hepatitis A vaccine and DMPC; and a combination of a melanoma vaccine and QS-21.

In addition to the above combinations of vaccines and adjuvants, examples of a preferable combination of drugs include: a combination of a hypotensive drug and a hypotensive diuretic agent such as a combination of lisinopril and hydrochlorothiazide, a combination of methyldopa and hydrochlorothiazide, a combination of clonidine hydrochloride and chlorthalidone, or a combination of benazepril hydrochloride and hydrochlorothiazide; a combination of antidiabetic agents such as a combination of insulin and metformin hydrochloride; and any other combination such as a combination of ozagrel hydrochloride and ozagrel sodium or a combination of codeine hydrochloride and promethazine hydrochloride.

In addition, multiple kinds of ionic drugs to be held by the electrode assembly for iontophoresis in the present invention may be appropriately combined depending on, for example, the kind of a disease and the condition of a patient. This means that different ionic drugs may be held by each electrode assemblies or multiple kinds of ionic drugs may be combined in a single electrode assembly.

The amount of an ionic drug is determined for each individual ionic drug in such a manner that an effective blood concentration preset upon application to a patient can be obtained for an effective time period. The amount is set by one skilled in the art in accordance with, for example, the size and thickness of a drug solution holding portion or the like, the area of a drug release surface, a voltage in an electrode device, and an administration time.

JP H03-146511 A, JP 2004-300292 A and WO 03/037425 A1 according to the applicant of the present invention describes details about the above-described respective components, and the contents described in the document are also included in the present invention.

## Claims

1. An electrode assembly for iontophoresis comprising an ionic drug, comprising at least:
an electrode connected to an electric power source device having the same polarity as that of the ionic drug in the electrode assembly;
an electrolyte solution holding portion impregnated with an electrolyte solution, the electrolyte solution holding portion being placed adjacent to the electrode;
an ion exchange membrane which is selectively permeable to an ion having a polarity opposite to that of the ionic drug, the ion exchange membrane being placed adjacent to the electrolyte solution holding portion;
a drug solution holding portion impregnated with the ionic drug, the drug solution holding portion being placed adjacent to the ion exchange membrane; and
an ion exchange membrane which is selectively permeable to an ion having the same polarity as that of the ionic drug, the ion exchange membrane being placed adjacent to the drug solution holding portion,
wherein a shape-memory separator capable of switching between passage of a substance and blocking of the substance owing to deformation of a shape-memory resin is placed adjacent to at least one surface of the ion exchange membrane which is selectively permeable to an ion having a polarity opposite to that of the ionic drug.

2. The electrode assembly for iontophoresis according to claim 1, wherein:
the separator is made of a membrane of the shape-memory resin capable of being porous or a porous membrane containing the shape-memory resin; and
the separator closes a pore to block movement of a substance during production or storage of the electrode assembly, and changes to be porous owing to a temperature change or voltage application, thereby allowing the substance to pass.

3. The electrode assembly for iontophoresis according to claim 2, wherein the separator blocks the passage of the substance at a temperature lower than 30°C and becomes porous owing to deformation of the shape-memory resin by heating at 30°C or higher, thereby allowing the substance to pass.

4. The electrode assembly for iontophoresis according to claim 2, wherein the separator becomes porous owing to deformation of the shape-memory resin by heating at 40°C or higher, thereby allowing the substance to pass, and then maintains a deformed state even when being cooled to a temperature lower than 40°C to thereby allow the substance to pass.

5. An iontophoresis device comprising:
an electric power source device;
a drug administration means comprising two or more electrode assemblies including one or more electrode assemblies according to claim 1, the drug administration means being connected to the electric power source device; and
a current control means for controlling a current flowing to the electrode assemblies,
wherein the ionic drug is released from the electrode assemblies to be administered to a living body transdermally in accordance with the current flowing from the current control means.

6. An electrode assembly for iontophoresis comprising an ionic drug, comprising at least:
an electrode connected to an electric power source device having the same polarity as that of the ionic drug in the electrode assembly;
an electrolyte solution holding portion impregnated with an electrolyte solution, the electrolyte solution holding portion being placed adjacent to the electrode;
an ion exchange membrane which is selectively permeable to an ion having a polarity opposite to that of the ionic drug, the ion exchange membrane being placed adjacent to the electrolyte solution holding portion;
a drug solution holding portion impregnated with the ionic drug, the drug solution holding portion being placed adjacent to the ion exchange membrane; and
an ion exchange membrane which is selectively permeable to an ion having the same polarity as that of the ionic drug, the ion exchange membrane being placed adjacent to the drug solution holding portion,
wherein a shape-memory separator capable of switching between passage of a substance and blocking of the substance owing to deformation of a shape-memory resin is placed adjacent to at least one surface of the ion exchange membrane which is selectively permeable to an ion having the same polarity as that of the ionic drug.
